Europäisches Patentamt

European Patent Office

Office européen des brevets

(19)

(11) Veröffentlichungsnummer: **0 224 748**
**B1**

(12) **EUROPÄISCHE PATENTSCHRIFT**

(45) Veröffentlichungstag der Patentschrift: **01.08.90**

(21) Anmeldenummer: **86115405.2**

(22) Anmeldetag: **06.11.86**

(51) Int. Cl.⁵: **C 07 C 11/22,** C 07 C 11/14,
C 07 C 7/11

(54) **Verfahren zum Gewinnen von Methylazetylen und Propadien.**

(30) Priorität: **07.11.85 DE 3539553**

(43) Veröffentlichungstag der Anmeldung:
**10.06.87 Patentblatt 87/24**

(45) Bekanntmachung des Hinweises auf die
Patenterteilung:
**01.08.90 Patentblatt 90/31**

(84) Benannte Vertragsstaaten:
**DE GB NL**

(56) Entgegenhaltungen:
**DE-A-2 149 184**
**US-A-3 314 218**

(73) Patentinhaber: **Linde Aktiengesellschaft
Abraham-Lincoln-Strasse 21
D-6200 Wiesbaden (DE)**

(72) Erfinder: **Haehn, Peter Clemens, Dipl.-Ing.
Rosenweg 52f
D-8192 Geretsried (DE)**

(74) Vertreter: **Schaefer, Gerhard, Dr.
Linde Aktiengesellschaft Zentrale
Patentabteilung
D-8023 Höllriegelskreuth (DE)**

Courier Press, Leamington Spa, England.

EP 0 224 748 B1

**Beschreibung**

Die Erfindung betrifft ein Verfahren zur Gewinnung von Methylazetylen und Propadien aus einem C$_3$-haltigen Einsatzstrom durch Wäsche mit einem physikalisch wirkenden Lösungsmittel, das nach Beladung mit Methalazetylen und Propadien regeneriert und wiederverwendet wird.

Die bei der Spaltung von Kohlenwasserstoffen zur Olefinherstellung erzeugten Spaltgase enthalten in geringen Mengen Methylazetylen und Propadien. Die Spaltgase werden üblicherweise nach Abtrennung von Schweröl- und Krackbenzinanteilen verdichtet und rektifikatorisch zerlegt. Dabei wird eine Roh-C$_3$-Fraktion gewonnen, die im wesentlichen (zu etwa 75 bis 95 mol%) aus Propylen besteht, daneben bis zu einigen Prozent die C$_3$-Azetylene Methylazetylen und Propadien sowie Propan enthält.

Gemäß DE-Patent 21 49 184 ist es möglich, aus einem derartigen Einsatzstrom durch Wäsche mit Dimethylformamid Methylazetylen und Propadien abzutrennen. Ziel des bekannten Verfahrens ist es, einerseits die C$_3$-Azetylene und andererseits ein von C$_3$-Azetylenen freies Propan/Propylengemisch zu gewinnen. Hierzu wird der Einsatzstrom mit Dimethylformamid als Lösungsmittel im Gegenstrom gewaschen, wobei das Lösungsmittel die C$_3$-Azetylene sowie einen Teil des Propans und Propylens aufnimmt. Das beladene Lösungsmittel wird nach Entspannung einem Stripper-Absorber zugeführt, der so betrieben wird, daß im Sumpf ein mit gleichen mol-Mengen C$_3$-Azetylen und Propylen/Propan beladenes Lösungsmittel abgezogen wird. Über Kopf kann damit ein im wesentlichen aus Propan/Propylen und geringen Mengen C$_3$-Azetylenen bestehendes Gasgemisch gewonnen werden.

Das beladene Lösungsmittel wird in einer nachfolgenden Regenerierkolonne vollständig von den mitgeführten Gasen befreit, die im gewünschten Verhältnis 50 mol% C$_3$-Azetylen und 50 mol% oder weniger Propan/Propylen abgezogen werden. Mit dem bekannten Verfahren ist es dabei auch möglich, vom Kopf der Regenerierkolonne reines oder angereichertes C$_3$-Azetylen und vom Kopf des Stripper-Absorbers C$_3$-azetylenfreies Propan/Propylen zu gewinnen.

Der Nachteil des bekannten Verfahrens besteht jedoch im wesentlichen darin, daß das Kopfprodukt aus dem Stripper-Absorber einen erheblichen Anteil der Gesamtgasmenge ausmacht und aus Wirtschaftlichkeitsgründen wiederverwendet werden muß. Hierzu muß dieses Rückführgas verdichtet werden. Andererseits muß Energie für die Verdichtung des Strippgases für den Stripper-Absorber aufgewendet werden. Da außerdem sehr große Waschmittelmengen umgewälzt werden, ist auch hierfür viel Energie aufzuwenden. Überdies ist mit dem bekannten Verfahren eine getrennte Gewinnung von Propadien und Methylazetylen nicht möglich.

Der vorliegenden Erfindung liegt daher die Aufgabe zugrunde, ein Verfahren der eingangs genannten Art so zu verbessern, daß eine getrennte Gewinnung von Propadien und Methylazetylen ermöglicht wird bei gleichzeitiger Reduzierung der Energiekosten.

Diese Aufgabe wird erfindungsgemäß dadurch gelöst, daß die Absorption von Methylazetylen und Propadien mit einem ersten Teilstrom eines Lösungsmittels bei erhöhtem Druck durchgeführt wird, daß aus dem beladenen ersten Teilstrom Propadien abgetrennt und mit einem zweiten Teilstrom desselben Lösungsmittels bei vermindertem Druck rückgewaschen wird, daß aus dem im wesentlichen mit Propadien beladenen zweiten Teilstrom Propadien abgetrennt und aus dem im wesentlichen mit Methylazetylen beladenen ersten Teilstrom Methylazetylen abgetrennt wird.

Als Einsatzstrom bei dem erfindungsgemäßen Verfahren wird das Sumpfprodukt eines Propan/Propylen-Splitters verwendet, in dem üblicherweise 20 bis 40 mol% C$_3$-Azetylene, 20 bis 50 mol% Propan und als Rest Propylen enthalten sind. Dieser Einsatzstrom wird mit einem organischen, physikalisch wirkenden Lösungsmittel, wie Dimethylformamid (DMF) oder N-Methylpyrrolidon (NMP) bei erhöhtem Druck, der zweckmäßig zwischen 2 und 20 bar, vorzugsweise zwischen 6 und 12 bar liegt, gewaschen, so daß ein im wesentlichen aus Propan und Propylen bestehendes Gasgemisch (<0,2% Methylazetylen+Propadien) als Kopffraktion des Absorbers gewonnen werden kann.

Das Lösungsmittel nimmt neben den C$_3$-Azetylenen auch Propan und Propylen auf. Um diese Gase freizusetzen ist vorgesehen, das beladene Lösungsmittel anzuwärmen, so daß Propan und Propylen aufgrund ihrer geringeren Löslichkeit im Lösungsmittel in die Gasphase übergehen. Die Anwärmung des Lösungsmittels kann dabei vorteilhaft im Wärmetausch zu vollständig regeneriertem Lösungsmittel, das bei erhöhter Temperatur vorliegt, oder Fremdwärme, durchgeführt werden. Die erwärmte propan-propylenhaltige Gasphase kann in einem Abscheider abgezogen und als Strippgas zur Absorption zurückgeführt werden, um das beladene Lösungsmittel aufzukonzentrieren, d.h. Propan und Propylen auszutreiben. Durch diese Maßnahme können die Restgasfraktionen nachfolgend noch zu beschreibender Stripper-Absorber-Kolonnen bei Niederdruck im Vergleich zum Absorberkopfprodukt sehr klein gehalten werden, beispielsweise bei 3 bis 6% des gesamten Propan/Propylenanteils des Einsatzgases.

Propadien und Methylazetylen besitzen unterschiedliche Löslichkeiten in DMF oder NMP. Aufgrund der unterschiedlichen Löslichkeiten ist es möglich, aus dem beladenen ersten Teilstrom Propadien freizusetzen. Dies kann bevorzugt in einem Stripper durch Einbringung von Wärme erfolgen. Dabei wird das Methylazetylen in Sumpf des Strippers aufkonzentriert und das Propadien über Kopf abgetrieben. Die Stripping des beladenen ersten Teilstromes im Sumpf der Strippkolonne kann soweit geführt werden, daß im Lösungsmittel nur noch hochreines Methylazetylen verbleibt. Dies ist mit vergrößertem Energieaufwand möglich. Besteht die Notwendigkeit zur Gewinnung von hochreinem Methylazetylen nicht, dann werden vorteilhafterweise 80 mol% des Methylazetylen und 50 mol% des Propadien im Lösungsmittel belassen.

Das mit Propadien stark angereicherte Kopfprodukt aus dem Stripper wird erfindungsgemäß mit einem zweiten Teilstrom desselben Lösungsmittels unter vermindertem Druck von vorzugsweise 1 bis 3 bar selektiv rückgewaschen. Als zweiter Teilstrom kann vorteilhaft ein Teilstrom von 30 bis 50 mol% des ersten Teilstromes verwendet werden. Diese erste Rest-$C_3$-Fraktion kann mengenmäßig aufgrund der erfolgten Vorstrippung sehr klein gehalten werden und ohne Zusatzverdichtung beispielsweise als Heizgas abgegeben werden. Der im wesentlichen mit Propadien beladene zweite Teilstrom wird in einem nachfolgenden Stripper/Absorber von den mitgeführten Inertgasen, Propan und Propylen getrennt. Auch diese zweite Rest-$C_3$-Fraktion kann mengenmäßig aufgrund der erfolgten Vorstrippung sehr klein gehalten werden und ohne Zusatzverdichtung beispielsweise als Heizgas abgegeben werden.

Nach dem erfindungsgemäßen Verfahren liegen nunmehr zwei Lösungsmittelströme vor, wobei der eine im wesentlichen mit Propadien und der andere im wesentlichen mit Methylazetylen beladen ist. Diese beiden Lösungsmittelströme können von Propadien bzw. Methylazetylen getrennt befreit werden, und zwar in bevorzugter Weise durch Strippen mit durch Aufkochen erzeugtem Dampf. Die Abtrennung von Propadien und/oder Methylazetylen erfolgt dabei bevorzugt bei einem Druck zwischen 1 und 3 bar. Dies gilt auch für die Abtrennung des Propadien aus dem beladenen ersten Teilstrom.

Die getrennte Rückgewinnung, d.h. Desorption/Absorption von Propadien und Methylazetylen ist aufgrund der unterschiedlichen Löslichkeiten der beiden $C_3$-Azetylenisomere in DMF oder NMP möglich.

Für den Fall, daß neben einer reinen Propadienfraktion keine reine, Methylazetylenfraktion, sondern ein aus Methylazetylen und Propadien bestehendes Kopfprodukt gewonnen werden soll, ist es nach einer Ausführungsform des erfindungsgemäßen Verfahrens möglich, von Propadien befreiten zweiten Teilstrom dem mit Methylazetylen beladenen ersten Teilstrom zuzumischen und zusammen mit diesem einer $C_3$-Azetylenabtrennung zuzuführen. Diese Verfahrensweise bietet den Vorteil, daß die Abtrennung des Propadien aus dem ersten Teilstrom nicht vollständig sein muß. Es kann vielmehr etwas Propadien und damit auch mitgeführtes Methylazetylen im zweiten Teilstrom verbleiben, da auch diese Restfraktionen in der Regenerierung als Produkt gewonnen werden. Wenn dabei eine sehr hohe Reinheit der $C_3$-Azetylenprodukte gefordert ist, hat es sich als zweckmäßig erwiesen, wenn das Kopfprodukt aus der letzten Regenerierkolonne mit Wasser gewaschen wird, um Lösungsmittel aus der gewonnenen Gasfraktion rückzuwaschen.

Gemäß einer weiteren besonders vorteilhaften Ausführungsform des erfindungsgemäßen Verfahrens kann das Abtrennen und Rückwaschen von Propadien in einer einzigen Kolonne durchgeführt werden, die dann allerdings mittels Kaminboden o.ä. in einen oberen und einen unteren Abschnitt unterteilt ist.

Mit dem erfindungsgemäßen Verfahren kann somit einerseits eine Rein-Propadien-Fraktion und andererseits entweder eine Rein-Methylazetylenfraktion oder ein Gemisch aus Methylazetylen und Propadien bei niedrigeren Betriebsmittel-Kosten erzeugt werden. Als Betriebsmittel werden nämlich nur Niederdruckdampf und Kühlwasser und in geringer Menge Mitteldruckdampf benötigt. Sehr geringe Kältemittelmengen finden Anwendung lediglich zur Verringerung von Lösungsmittelverlusten.

Im folgenden sei die Erfindung anhand eines schematisch dargestellten Ausführungsbeispiels näher erläutert.

Über Leitung 1 werden 4684 kg/h eines Einsatzstromes herangeführt. Der Einsatzstrom ist der Sumpfprodukt eines $C_3$-Splitters und hat folgende Zusammensetzung:

    31 mol% Methylazetylen+Propadien
    24 mol% Propan
    45 mol% Propylen.

Der Einsatzstrom fällt flüssig an und wird daher zunächst in einem Verdampfer 2 verdampft. Der Einsatzstrom wird sodann mit einer Temperatur von 24°C in den unteren Teil eines Absorbers 3 geleitet und im Gegenstroms zu über Leitung 4 herangeführtem regeneriertem Lösungsmittel (23 700 kg/h DMF mit einer Temperatur von 32°C) gewaschen. In der Absorptionskolonne 3 herrscht dabei ein Druck von 9 bar. Das praktisch $C_3$-azetylenfreie Kopfprodukt wird mit Hilfe von einem Kältemittel 5 zur Auskondensation von DMF-Dämpfen geringfügig auf 18°C abgekühlt und in einer Menge von 3093 kg/h über Leitung 6 abgegeben. In diesem Kopfprodukt sind weniger als 0,5 mol% $C_3$-Azetylene enthalten.

Das beladene DMF wird im Sumpf des Absorbers 3 mit etwa 150°C warmen Propan/Propylen-Dämpfen zur Aufkonzentration des $C_3$-Azetylens in der DMF-Phase und zum Austreiben von Propan/Propylen gestrippt.

Die Propan/Propylen-Dämpfe werden dabei auf folgende Weise erzeugt. Das beladene DMF verläßt den Absorber über Leitung 7 mit einer Temperatur von 82°C. In einem Wärmetauscher 8 wird das beladene Lösungsmittel zu über Leitung 9 herangeführtem, vollständig regeneriertem Lösungsmittel (37 400 kg/h) auf etwa 150°C angewärmt. Bei dieser Anwärmung werden die mitgelösten Inertgase Propan und Propylen freigesetzt und können in einem Abscheider 10 über Leitung 11 abgezogen und als Strippgas für den Sumpf des Absorbers verwendet werden.

Aus dem Abscheider 10 werden überdies über Leitung 12 25274 kg/h beladene DMF abgezogen. Die beladene DMF gibt ihre Wärme in einem Reboiler 13 einer nachfolgenden Strippkolonne 14 ab, wobei sie sich auf 114°C abkühlt. Nach weiterer Abkühlung gegen Kühlwasser in einem Kühler 15 auf 54°C wird die beladene DMF in den oberen Teil des bei 2 bar betriebenen Strippers 14 gegeben und mit dem im Reboiler erzeugten Dampf gestrippt, wobei 577 kg/h einer Gasfraktion abgetrennt werden, die neben Propadien auch Propan, Propylen und Methylazetylen enthält.

Diese Gasfraktion steigt mit einer Temperatur von 54°C nach oben und gelangt über einen Kaminboden 16 in einen in der gleichen Kolonne angeordneten Absorberteil 17. Dort wird die Gasfraktion mit 8200 kg/h DMF, die über Leitung 18 vom regenerierten Lösungsmittel aus Leitung 4 abgezweigt wird, bei einem Druck von 1,8 bar gewaschen. Über Leitung 19 verlassen 64 kg/h Propan/Propylen den Absorberteil mit einer Temperatur von 35°C.

Der im wesentlichen mit Propadien beladene zweite Teilstrom (8700 kg/h) wird über Leitung 20 vom unteren Teil des Absorbers mit einer Temperatur von 46°C abgezogen und einer bei 1,8 bar betriebenen Stripper/Absorberkolonne 21 zugeführt. In der Kolonne 21 werden mit in einem Reboiler 22 mittels Dampf erzeugtem Strippdampf die Inertgasanteile Propan und Propylen aus der DMF ausgetrieben. Zur Erhöhung der Propadienausbeute werden über Leitung 23 5500 kg/h regenerierte DMF auf den oberen Teil der Kolonne 21 aufgegeben. Über Leitung 24 verlassen 37 kg/h des restlichen Inertgasanteils, wie Propan/Propylen zusammen mit Propadien mit einer Temperatur von 36°C den Stripper.

Von Sumpf der Kolonne 21 werden über Leitung 25 14,2 t/h ein im wesentlichen mit Methylazetylen beladener zweiter Teilstrom (78°C) abgezogen und zusammen mit 24,7 t/h mit Methylazetylen beladenem erstem Teilstrom 13a (86°C) aus dem Stripper 14 in einem Wärmetauscher 26 gegen Niederdruckdampf auf 109°C angewärmt und einer Regenerierkolonne 27 aufgegeben. Dort wird die DMF mit im Reboiler durch Mitteldruckdampf erzeugtem Strippdampf gestrippt. Die reine DMF kann mit einer Temperatur von 169°C über Leitung 9 abgezogen und nach Abkühlung im Wärmetauscher 8 auf 93°C über Leitung 28 mit Pumpe 29 in einem Kühler 30 auf 32°C abgekühlt und über Leitung 4 wieder auf die Absorber 3, 17 und Stripper/Absorberkolonne 21 aufgegeben werden.

Über Kopf werden über Leitung 31 aus der Regenerierkolonne 27 nach Abkühlung auf −8°C 551 kg/h Propadien, 790 kg/h Methylazetylen sowie 8 kg/h Lösungsmitteldämpfe abgezogen. Um hochreines C₃-Azetylenprodukt zu gewinnen, wird diese Kopffraktion der Regenerierkolonne 27 einem Wäscher 32 zugeführt, dem über Leitung 33 Waschwasser aufgegeben wird. Über Leitung 34 kann somit DMF-freies C₃-Azetylen gewonnen werden. Mit DMF beladenes Wasser wird über Leitung 35 abgezogen.

Betriebsmittel

|   |   |   |
|---|---|---|
| Niederdruckdampf | 1,8 t/h |
| Mitteldruckdampf | 3 t/h |

Kühlwasserbedarf bei einer Änwärmung um 10°C 200 t/h. Bei Anwendung von Luftkühlern kann der Kühlwasserbedarf typischerweise auf 100 t/h verringert werden.

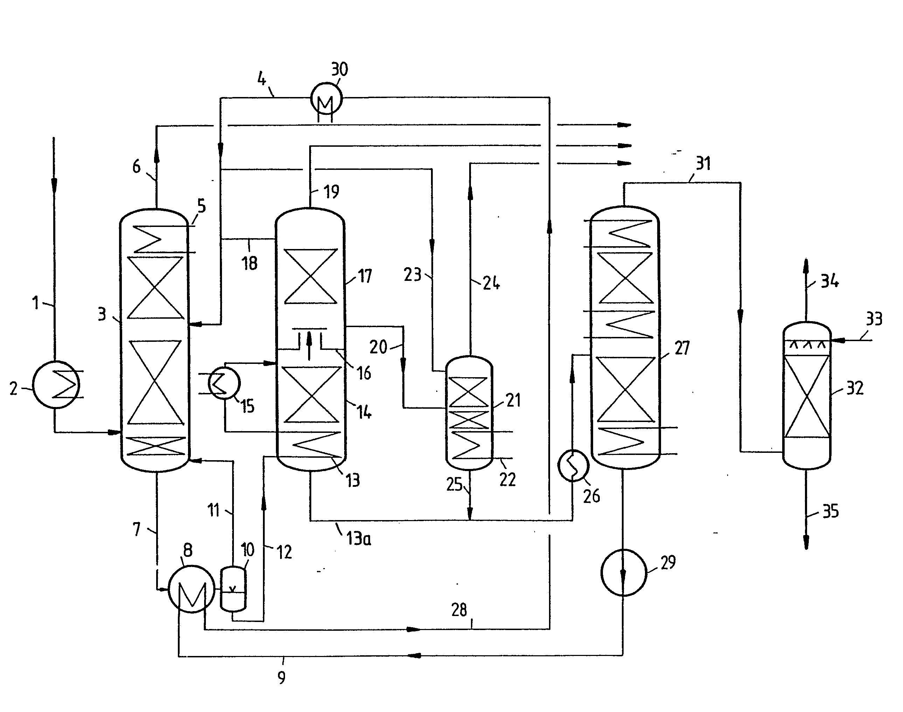

**Patentansprüche**

1. Verfahren zum Gewinnen von Methylazetylen und Propadien aus einem C₃-haltigen Einsatzstrom durch Wäsche mit einem physikalisch wirkenden Lösungsmittel, das nach Beladung mit Methylazetylen und Propadien regeneriert und wiederverwendet wird, dadurch gekennzeichnet, daß die Absorption (3) von Methylazetylen und Propadien mit einem ersten Teilstrom eines Lösungsmittels (4) bei erhöhtem Druck durchgeführt wird, daß aus dem beladenen ersten Teilstrom (12) bevorzugt Propadien abgetrennt und dieses mit einem zweiten Teilstrom desselben Lösungsmittels (18) bei vermindertem Druck rückgewaschen wird (17), daß aus dem im wesentlichen mit Propadien beladenen zweiten Teilstrom (20) Propadien abgetrennt und aus dem im wesentlichen mit Methylazetylen beladenen ersten Teilstrom (13a) Methylazetylen abgetrennt wird.

2. Verfahren nach Anspruch 1, dadurch gekennzeichnet, daß die Abtrennung von Propadien und/oder Methylazetylen durch Strippen durchgeführt wird.

3. Verfahren nach Anspruch 1, dadurch gekennzeichnet, daß die Absorption mit dem ersten Teilstrom des Lösungsmittels bei einem Druck zwischen 2 und 20 bar, vorzugsweise 6 und 12 bar und mit dem zweiten Teilstrom bei einem Druck zwischen 1 und 3 bar durchgeführt wird.

4. Verfahren nach Anspruch 1 oder 2, dadurch gekennzeichnet, daß die Abtrennung von Propadien und/oder Methylazetylen bei einem Druck zwischen 1 und 3 bar durchgeführt wird.

5. Verfahren nach einem der Ansprüche 1 bis 4, dadurch gekennzeichnet, daß der im wesentlichen mit Propadien beladene zweite Teilstrom dem im wesentlichen mit Methylazetylen beladenen ersten Teilstrom zugeführt wird.

6. Verfahren nach Anspruch 1, dadurch gekennzeichnet, daß der zweite Teilstrom 30 bis 50% des ersten Teilstroms beträgt.

7. Verfahren nach einem der Ansprüche 1 bis 6, dadurch gekennzeichnet, daß der beladene erste Teilstrom vor der Propadienabtrennung angewärmt wird.

8. Verfahren nach Anspruch 7, dadurch gekennzeichnet, daß die Anwärmung des beladenen ersten Teilstroms im Wärmetausch zu vollständig regeneriertem Lösungsmittel durchgeführt wird.

9. Verfahren nach Anspruch 7 oder 8, dadurch gekennzeichnet, daß das nach der Anwärmung des beladenen ersten Teilstroms gewonnene Gas abgetrennt und als Strippgas im Gegenstrom mit dem beladenen ersten Teilstrom im Sumpf der Absorberkolonne gewaschen wird.

10. Verfahren nach Anspruch 1, dadurch gekennzeichnet, daß das Abtrennen von Propadien aus dem ersten Teilstrom und anschließende Rückwaschen von Propadien in einer Kolonne durchgeführt wird.

11. Verfahren nach einem der Ansprüche 1 bis 5, dadurch gekennzeichnet, daß das Kopfprodukt einer jeden Regenerierung der mit Propadien und/oder Methylazetylen beladenen Lösungsmittel zur Befreiung der Produkte vom Lösungsmittel mit Wasser gewaschen wird.

12. Verfahren nach Anspruch 5, dadurch gekennzeichnet, daß Methylazetylen und Propadien getrennt voneinander aus dem mit Methylazetylen und Propadien beladenen Lösungsmittel abgestrippt und als getrennte Produkte gewonnen werden.

## Revendications

1. Procédé pour l'obtention de méthylacétylène et de propadiène à partir d'un courant contenant une charge en $C_3$ par lavage avec un solvant à action physique, lequel est régénéré et réutilisé après avoir été chargé de méthylacétylène et de propadiène, caractérisé en ce que l'absorption (3) du méthylacétylène et du propadiène est effectuée par un premier courant partiel d'un solvant (4) sous un pression élevée, que l'on sépare préférentiellement de ce permier courant partiel (12) chargé du propadiène et qu'on lave celui-ci par un deuxième courant partiel du même solvant (18) à une pression plus réduite (17), en ce que l'on sépare le propadiène de ce deuxième courant partiel (20) chargé essentiellement de propadiène et en ce que l'on sépare le méthylacétylène du premier courant partiel (13a) essentiellement chargé de méthylacétylène.

2. Procédé selon la revendication 1, caractérisé en ce que la séparation du propadiène et/ou du méthylacétylène est effectuée par stripping.

3. Procédé selon la revendication 1, caractérisé en ce que l'absorption par le premier courant partiel du solvant est effectuée à une pression comprise entre 2 et 20 bars, de préférence 6 et 12 bars, et est effectuée par le deuxième courant partiel sous une pression comprise entre 1 et 3 bars.

4. Procédé selon la revendication 1 ou 2, caractérisé en ce que la séparation du propadiène et/ou du méthylacétylène est effectuée sous une pression comprise entre 1 et 3 bars.

5. Procédé selon l'une des revendications 1 à 4, caractérisé en ce que le deuxième courant partiel chargé essentiellement de propadiène est ajouté au premier courant partiel chargé essentiellement de méthylacétylène.

6. Procédé selon la revendication 1, caractérisé en ce que le deuxième courant partiel représente 30 à 50% du premier courant partiel.

7. Procédé selon l'une des revendications 1 à 6, caractérisé en ce que le premier courant partiel chargé est réchauffé avant la séparation du propadiène.

8. Procédé selon la revendication 7, caractérisé en ce que le réchauffage du premier courant partiel chargé est effectué par échange thermique avec le solvant complètement régénéré.

9. Procédé selon la revendication 7 ou 8, caractérisé en ce que le gaz obtenu après réchauffage du premier courant partiel chargé est séparé et constitue un gaz d'entraînement qui est lavé à contre-courant du premier courant partiel chargé en cuve de la colonne d'absorption.

10. Procédé selon la revendication 1, caractérisé en ce que la séparation du propadiène à partir du premier courant partiel et le lavage à contre-courant subséquent du propadiène sont effectuées dans une colonne.

11. Procédé selon l'une des revendications 1 à 5, caractérisé en ce que le produit de tête de chacun des solvants chargé de propadiène et/ou de méthylacétylène des régénérations est lavé à l'eau pour libérer les produits entraînés par le solvant.

12. Procédé selon la revendication 5, caractérisé en ce que méthylacétylène et propadiène sont séparés est entraînement du solvant chargé de méthylacétylène et de propadiène et sont obtenus en tant que produits distincts.

## Claims

1. A process for reclaiming methylacetylene and propadiene from an input stream containing $C_3$ by scrubbing with a physically active solvent which, having been charged with methylacetylene and propadiene is regenerated and re-used, characterised in that the absorption (3) of methylacetylene and propadiene is carried out using a first sub-stream of a solvent (4) at an increased pressure, that preferably propadiene is separated from the charged, first sub-stream (12) and is rescrubbed (17) using a second sub-stream of the same solvent (18) at reduced pressure, that propadiene is separated from the second sub-stream (20) which is fundamentally charged with propadiene, and methylacetylene is separated from the first sub-stream (13a) which is fundamentally charged with methylacetylene.

2. A process as claimed in Claim 1, characterised in that the separation of propadiene and/ or methylacetylene is carried out by stripping.

3. A process as claimed in Claim 1, characterised in that the absorption using the first sub-stream of the solvent is carried out at a pressure of between 2 and 20 bar, preferably 6 and 12 bar, and the absorption using the second sub-stream is carried out at a pressure of between 1 and 3 bar.

4. A process as claimed in Claim 1 or 2, characterised in that the separation of propadiene and/or methylacetylene is carried out at a pressure of between 1 and 3 bar.

5. A process as claimed in one of the Claims 1 to 4, characterised in that the second sub-stream which is fundamentally charged with propadiene is fed to the first sub-stream which is fundamentally charged with methylacetylene.

6. A process as claimed in Claim 1, characterised in that the second sub-stream amounts to 30 to 50% of the first sub-stream.

7. A process as claimed in one of the Claims 1 to 6, characterised in that the charged, first sub-stream is heated prior to the separation of the propadiene.

8. A process as claimed in Claim 7, characterised in that the heating of the charged, first sub-stream is carried out in heat exchange with fully regenerated solvent.

9. A process as claimed in Claim 7 or 8, characterised in that the gas obtained after the heating of the charged, first sub-stream is separated and as stripping gas is scrubbed in the counter-current direction to the charged, first sub-stream in the sump of the absorber column.

10. A process as claimed in Claim 1, characterised in that the separation of propadiene from the first sub-stream and the subsequent rescrubbing of propadiene is carried out in one column.

11. A process as claimed in one of the Claims 1 to 5, characterised in that the head product of each regeneration of the solvents charged with propadiene and/or methylacetylene is scrubbed with water in order to free the product from the solvent.

12. A process as claimed in Claim 5, characterised in that methylacetylene and propadiene are stripped separately from one another from the solvent charged with methylacetylene and propadiene and are obtained as separate products.

EP 0 224 748 B1